# EUROPEAN PATENT APPLICATION

(11) **EP 1 106 188 A1**
(43) Date of publication of application: **13.06.2001**
(21) Application number: 00126723.6
(22) Date of filing: 05.12.2000
(51) Int. Cl.: A61L 2/04, A61L 2/08, A61L 2/10, F24J 2/34, C02F 1/14

(54) **Plastic containers for solar disinfection of water**

(30) Priority: 09.12.1999 US 169755 P
(71) Applicant: SOCIETE DES PRODUITS NESTLE S.A., 1800 Vevey (CH)
(72) Inventor: Walker, Carey, 1801 Le Mont-Pelerin (CH); Frangsmyr, Brita, New Milford, CT 06776 (US)

(57) **Abstract**

A container is provided for the disinfection of drinking water. The container comprises a body having a first and a second side and defining an interior for receiving a fluid. The first side being defined, at least in part, by a UV transparent layer. The second side is defined, at least in part, by a second layer that either reflects sunlight or generates heat in response to solar energy.

## Description

### BACKGROUND OF THE INVENTION

The present invention relates generally to methods and devices for disinfecting drinking water.

There are a number of diarrheal diseases that result from drinking or using contaminated water. These diseases are major contributors to morbidity and mortality in developing countries. It is estimated that as many as six million deaths per year can be attributed to such diseases. Thielman, N.M. and R.L. Geurrant, *"From Rwanda to Wisconsin: The Global Relevance of Diarrhoeal Diseases",* The Journal of Microbiology; 44:155-156 (1996).

In the last two decades, a significant amount of research has focused on the development of systems for decontaminating water in developing countries. Because sunlight is considered the single most important contributor to bacterial die-off in natural water supplies, and is freely available, many efforts have focused on utilizing this resource for disinfecting water supplies.

Solar radiation is not evenly distributed throughout the world. Moreover, it is depleted and attenuated by the atmosphere. Only a fraction of UV-B (280-320 nm) and UV-A (320-400 nm) radiation, approximately 4% reaches ground level. The most favorable belt for UV exposure is the northern hemisphere (15-35°). This area encompasses many developing nations located in northern Africa and the southern parts of Asia.

Solar box cookers (SBCs) have been designed for use in resource-poor areas. These devices simply harness the heating capacity of sunlight to raise the temperature of water to 65°C typically for a minimum of one hour. Usually, the heat that is generated is sufficient to pasteurize naturally contaminated water. Ciochetti, D.A., and R.H. Metcalf, *"Pasteurization of Naturally Contaminated Water With Solar Energy",* Applied Environmental Microbiology, 47:223-228 (1984). Such a system was deployed in Zambia following an outbreak of cholera, and reduced the bacterial load by 10⁴ CFU/ml water at 65°C, and effectively sterilized the water at 80°C, although the capacity was reduced at the higher temperature. Jorgensen, A.J. and F.K. Nohr, *"Cholera: Water Pasteurization Using Solar Energy",* Trop Doctor, 25:175 (1995).

Studies have been conducted in which either polyethylene bags (placed on a black or steel surface) or PET soft drink bottles (clear or partially blackened) were filled with river or pond water, inoculated with fecal coliforms and *Vibrio cholerae,* and placed in the sun. Bacterial viability was reduced by 3.0 logs after 140 minutes storage in plastic bags and the effect was dependent upon both heating and UV radiation. Sommer, et al., *"SODIS - An Emerging Water Treatment Process",* Aqua, 46:127-137, 1997. See also Conroy, R.M., et al., *"Solar Disinfection of Drinking Water and Diarrhoeal in Maasai Children: A Controlled Field Trial",* Lancet 348:1695-1697 (1996); Davies, C.M. et al., *"Sunlight and the Survival of Enteric Bacteria in Natural Waters",* J. Appl. Bacteriol. 70:265-274 (1991); McGuigan, K.G., et al., *"Solar Disinfection of Drinking Water Contained in Transparent Plastic Bottles: Characterizing the Bacterial Inactivation Process",* J. Appl. Microbiol. 84:1138-1148 (1998); and Reed, R.H., *"Solar Inactivation of Faecal Bacteria in Water: The Critical Role of Oxygen",* Lett. Appl. Microbiol. 24:276-280 (1997). Additionally, solar heating devices have been used to disinfect water. See Jorgensen, A.J.F. et al., *"Decontamination of Drinking Water by Direct Heating in Solar Panels", J.* Appl. Microbiol. 85:441-447 (1998).

Unfortunately, the use of plastic bags or bottles does not provide an optimum system for the disinfection of water in a relatively short time frame. Moreover, solar heating devices that have been employed have the disadvantage that they are complicated and expensive.

Accordingly, there is a need for an improved, simple method and device for the disinfection of water.

### SUMMARY OF THE INVENTION

The present invention provides methods and devices for disinfecting water. The devices and methods can be used as a primary means for disinfecting water or as adjunct to other means, e.g., boiling water.

To this end, in an embodiment, a container is provided for the disinfection of drinking water. The container comprises a body having a first and a second side and defining an interior for receiving water. The first side being defined, at least in part, by a UV transparent layer. The second side is defined, at least in part, by a second layer that generates heat in response to solar energy or amplifies the effect of light.

In an embodiment, the container includes a member for receiving water.

In a further embodiment, the member is a resealable fitment.

In a further embodiment, the second layer is constructed from a reflective plastic that amplifies the effect of light.

In a further embodiment, the second layer is constructed from a metalized reflective plastic.

In an embodiment, the second layer is constructed from a material that absorbs solar radiation. In a further embodiment, the second layer has a black surface.

In an embodiment, the second layer is not transparent to UV light.

In an embodiment, the body is made of a flexible plastic.

In another embodiment of the present invention, a method of reducing bacteria contaminates in water is provided. The method includes the steps of: providing a container having the body including a first side and a second side the first side is defined, at least in part, by a UV transparent layer, and the second side is defined, at least in part, by a second layer that generates heat in response to UV light; placing water in the container; and exposing the container to sunlight.

In an embodiment of the method, the container is exposed to sunlight for at least five hours and preferably for at least eight hours.

An advantage of the present intention is to provide a device for disinfecting water. Moreover, an advantage of the present invention is to provide an improved method for disinfecting water.

Further, an advantage of the present invention is to provide a reusable device for disinfecting water.

Still, an advantage of the present invention is to provide an inexpensive device and method for disinfecting water.

Furthermore, an advantage of the present invention is to provide a device and method for enhancing the safety of drinking water.

Additional features and advantages of the present invention are set forth in and will be apparent from the detailed description of the presently preferred embodiment and the figures.

### BRIEF DESCRIPTION OF THE FIGURES

Figure 1 illustrates a perspective view of an embodiment of the container of the present invention.

Figure 2 illustrates a cross sectional view of the container taken along lines II-II.

Figure 3 illustrates a cross sectional view of the container taken along lines III-III.

Figure 4 illustrates a cross sectional view of another embodiment of a portion the container of the present invention.

Figure 5 illustrates the effect of sunlight on the survival of *Escherichia coli* ATCC 25922 in 250 ml water contained in a plastic pouch (14X17 cm).

Figure 6 illustrates graphically the effect of sunlight on the survival of *Escherichia coli* ATCC 25922 in 1.0 liter of water contained in a plastic pouch (20 X 28 cm).

Figure 7 illustrates graphically the effect of sunlight on the survival of *staphylococcus aureus* 485 FDA in 1.0 liter of water contained in a plastic pouch (20 X 28 cm).

Figure 8 illustrates graphically the effect of sunlight on the survival *of Salmonella choleraesuis* subsp. *choleraesuis* serov. *typhimurium* in water (1 L) contained in a plastic pouch.

Figure 9 illustrates graphically the effect of sunlight on the survival *of Shigella sonnei* in 1.0 liter of water contained within a plastic pouch (on an overcast day) when the external temperature averaged 10C.

Figure 10 illustrates graphically the impact of container and beta-carotene on the solar disinfection of water containing *Escherichia coli* ATCC 25922 (reflective plastic pouch).

### DETAILED DESCRIPTION OF THE PRESENTLY PREFERRED EMBODIMENTS

The present invention provides improved containers and methods for sterilizing water.

Bacterial disinfection is dependent upon both UV and viable components (400-490 nm) of sunlight, which damage DNA and multiple cellular biomolecules, respectively. The effectiveness of solar disinfection methods and the ability of bacteria to enter into a viable, but nonculturable state, are influenced by salinity and the presence of UV absorbing components like humic acids. The photoinactivation of bacteria is markedly sensitive to aeration rate and sunlight is most effective in fully oxygenated water. Applicants believe that solar disinfection methods rely, at least in part, on the generation of reactive oxygen species and that the disinfection efficiency can be enhanced by the inclusion of the aeration step.

Applicants have discovered that the effective disinfection of water using solar energy can be achieved by using specifically designed plastic containers (pouch). In a preferred embodiment, the container includes two different faces or surfaces. One of the faces includes a UV transmitting layer. The second face or surface is either a reflective, e.g., metalized, plastic to amplify the light effect, or an absorptive, e.g., black, plastic.

Referring now to Figure 1, an embodiment of the container 10 of the present invention is illustrated. The container includes a body 12 that, in the preferred embodiment illustrated, is sealed along four sides 14, 16, 18, and 20. Of course, there are a variety of ways to fabricate a plastic container and therefore it is not necessary that the body 12 includes four side seals.

The body 10 includes a first surface 22 that includes a UV transmitting layer. The UV transmitting layer, as the name implies, provides a structure that allows a majority of ambient sunlight to be transmitted through the surface 22. A number of plastics can be used to construct the first surface 22. For example, the following materials can be used to construct the UV transmitting layer: ethylene vinyl acetate; polypropylene; polyethylene; polyvinyl chloride; polybutylene; polyvinylidene chloride; PET; and blends and combinations thereof. A great variety of plastics can be used. What is required is that the material does not contaminate the fluid contained therein and is substantially transparent to visible and UV light.

Referring to Figure 2, a cross-sectional view of the first surface 22 is illustrated. As illustrated, a three layer plastic structure 24 defines the first surface 22. The structure 24 includes an inner layer 26, a tie layer 28, and an outer layer 30. In a preferred embodiment, the outer layer 30 is biaxially oriented nylon, the tie layer 28 an adhesive, and the inner layer 26 is linear low density polyethylene. It should be appreciated that although in the preferred embodiment illustrated in Figure 1 at least substantially the entire first surface 22 is UV transmitting that the first surface can include non UV transmitting portions.

The first surface 22 is bonded, in the preferred embodiment illustrated in Figure 1, to a second surface 32 that includes a metalized reflective plastic to amplify the light effect. This allows light to pass through the first surface 22, through the container, and to be reflected off the second surface 32 back into and through the interior of the container 10.

Similar to the UV transmitting layer, a variety of plastics and materials can be used to construct the second surface 32. For example, the following plastics can be used: ethylene vinyl acetate; polypropylene; polyethylene; polyvinyl chloride; polyvinylidene chloride; polybutylene; PET; blends and combinations thereof can be used with a metalized layer.

Referring to Figure 3, an embodiment of the second layer 32 is illustrated in cross-section. The second layer 32 comprises, in the illustrated embodiment, a five layer structure. This structure includes an outer layer 33, core layer 35, and an inner layer 37. Two tie layers 34 and 36 are also provided. In the preferred embodiment illustrated the outer layer 33 is PET, the core layer 35 metalized PET, and the inner layer linear low density polyethylene 37. The two tie layers 34 and 36 are adhesives.

In an embodiment of the container, a portion of which is illustrated in Figure 4, the UV transmitting layer is bonded to a second surface 40 that includes an absorptive black plastic. This material will absorb light that passes through the interior and functions to elevate the temperature of the water contained in the container. The second layer can be constructed from a variety of plastics including ethylene vinyl acetate, polypropylene, polyethylene, polyvinyl chloride, polyvinylidene chloride, polybutylene, PET, blends and combinations thereof, with a black or dark ink added thereto. The second layer 40 in the illustrated embodiment, includes a sealant layer 42 (low density polyethylene), tie layer 44 adhesive, and an outer layer 46 PET to which black ink has been added.

In the preferred embodiment of the container 10 illustrated in Figure 1, the container 10 includes a fitment 48. The fitment 48 allows one to access the interior of the container 10. This allows water to be added to the container 10. Preferably the fitment 48 includes a removable closure 50 that allows an opening, defined by the fitment, to be closed after the container is filled sealing the container.

It should be noted that, there are a variety of different container structures that can be used in the present invention. Likewise, a variety of different techniques and materials can be used to construct the container.

By way of example, and not limitation, examples of the present invention will now be given.

### EXPERIMENT

The purpose of this experiment was to evaluate means of disinfecting microbiologically contaminated water through the transmission and amplification of incident solar radiation. Specifically, this experiment was to test the concept of a reusable plastic pouch designed to minimize the light path and assembled of materials selected to transmit and reflect the UV-A and UV-B radiation through a layer of water.

### Methodology, Trials, Results

Plastic pouches were designed to maximize the exposure of artificially contaminated Poland Springs Water to incident solar radiation. The pouches were of such dimensions as to contain 250 or 1000 ml of water at a depth (light path) of no more than 4.0 cm. A plastic film was selected to transmit a significant portion of the incident UV radiation above 250 nm and bonded to either a reflective or absorptive layer. Following the addition of sterile water, the pouches were heat-sealed and a bacterial load added (6.5 log CFU/ml) by injection through a rubber septum.

The plastic pouches were held in darkness (control samples) or placed in a foil lined cardboard box and exposed to sunlight (experimental samples) or exposed directly on a flat surface. Internal temperatures were monitored and samples were withdrawn periodically for an estimation of viable bacterial load.

In the first trial, the viable count of *Escherichia coli* was reduced 4.5 logs in 5 hours of exposure. In the first 1.5 hours, the reflective pouch was 3 logs more efficient even though the temperature in the absorptive pouch was 15°C higher.

In the second trial, *E. coli* viable counts again dropped 6.5 logs in 5 hours. There was no difference in bactericidal activity in the two pouches even though there was a 10°C difference in temperature.

In the second trial, viable counts *of Staphylococcus aureus* also declined 6.5 logs, and the reflective pouch was slightly more efficient.

In the third trial, *Salmonella typhimurium* viable counts declined 3.5 logs in the reflective pouch and only 1.0 log in the absorptive pouch. The day length had begun to decline by this experiment.

In the fourth trial, while *Shigella sonnei* counts declined 4.5 logs in the reflecting pouch, no effect was observed in the absorptive pouch. At the time of this experiment the ambient temperature averaged 10°C and the sky was considerably overcast.

In the final experiment, *E. coli* viable counts were reduced 7.0 logs in the reflective pouch, regardless of whether the trial was conducted in a reflective box or on the exposed roof surface. The addition of β-carotene, significantly reduced the bactericidal effect on sunlight, either by blocking sunlight or by absorbing oxygen, reducing the production of free radicals.

### Conclusion

Based upon these trials, the concept of a reusable plastic pouch that utilizes and amplifies the UV radiation of sunlight to sanitize water appears to be effective against a range of potential food pathogens including *E. coli, S. aureus, S. typhimurium* and *S. sonnei* when these organisms were inoculated into mineral water.

### Materials and Methods

The initial work involved designing a plastic pouch in which a UV transmitting layer is bonded to one of two surfaces: a) metalized, reflective plastic to amplify the light effect; or b) an absorptive, black plastic to elevate the water temperature. The reflective pouch consisted of two layers: a) a transparent layer of 60 ga biaxially oriented nylon (for strength), adhesive, and 3 mil linear low density polyethylene (for sealing) (see Fig. 2); and b) a reflective layer of 48 ga PET (for strength), adhesive, metalized 48 ga PET, adhesive, and 3 mil linear low density polyethylene (for sealing) (see Fig. 3). The absorptive pouch consisted of an identical transparent layer bonded to an absorbent layer of 48 ga PET/ ink (100% black)/adh/3 mil linear low-density polyethylene (for sealing) (see Fig. 4). The pouch materials were cut to the dimensions 14 X 17cm (250 ml) or 20 X 28cm (1.0 L), sealed on three sides, and filled volumetrically so that the light path did not exceed 4.0 cm.

The design of the reflective plastic pouch is illustrated in Figure 1. For most experiments the various plastic pouches were shielded from the wind by placing them within a three-sided cardboard box lined with aluminum foil. During the day the boxes were periodically realigned with the sun to maximize exposure to sunlight.

With the exception of the final set, all experiments involved the placement of the various plastic pouches on the roof of a facility in Connecticut, in a cardboard box that was lined with aluminum foil to increase reflectivity. Each pouch contained 250 ml or 1.0-L sterile water (tap or Poland Spring) which was inoculated (6.5 Log CFU/ml) with an overnight culture of one of the following: *Escherichia coli* ATCC 25922, *Staphylococcus aureus 485* FDA, *Salmonella choleraesuis* subsp. *choleraesuis* serov. *typhimurium,* or *Shigella sonnei.*

At each sample period, the temperature was recorded by thermocouple and an aliquot of contaminated water withdrawn by syringe; serially diluted and evaluated for viable bacteria on plate count agar (PCA) and/or Brain Heart Infusion Agar (BHI Agar). Only reflective pouches were used in the final set of experiments in which two were placed in the standard reflective box, one with β-carotene as an antioxidant (Reflective+-(β-carotene) and one without (Reflective), one was placed in a simple cardboard box with no reflective lining (Non-reflective), one was placed flat on the roof surface (unprotected) and one was covered (Control).

### Results and Discussion

In the initial trial using small pouches containing 250 ml volumes, the viable (culturable) colony forming units (CFU) per ml of *E. coli* contaminated water were reduced at least 4.5 logs during 5 hours of exposure to sunlight (see Figure 5). Though the final cell counts were comparable between the reflective and absorptive pouches, the reflective pouch proved 3 logs more efficient in the first hour and a half, even though the temperature in the absorptive pouch was 15°C higher.

More dramatic declines in *E. coli* viability were observed in the second trial, when a larger pouch volume (1.0 L) was employed. Detectable CFU/ml were reduced by 6.5 logs in 5 hours in pouches bearing reflective and absorptive backing (see Figure 6) which provided an effective sterilization. Even though an increase in temperature was observed with the absorptive pouch (45°C) over that obtained in the reflective pouch (34°C), terminal effectiveness was not improved. During the same trial a similar decrease in *S. aureus* viability was observed (6.5 log in 5 hours) (see Figure 7). Unlike the results obtained with *E. coli,* the viability of the *S. aureus* bacteria added to the reflective pouch declined more rapidly (≥ 1.0 log), indicating a higher level of UV sensitivity.

Additional studies were conducted with water-borne bacteria that have previously been implicated as etiologic agents in human diarrheal disease. The first of these, *Salmonella choleraesuis* subsp. *typhimurium,* also proved sensitive to solar radiation. In a little over 5 hours, viable CFU/ml declined no more than 1.0 log in the absorptive plastic pouch (35°C Max). The increased effectiveness of the reflective pouch was clearly evident (≥ 3.5 log reduction), though the absolute rate of killing was lower than that observed with *E. coli* and *S. aureus* (see Figure 8). This reduction in effectiveness could have resulted from a decrease in sunlight since the experiment was conducted later in the month of October and the sun was lower on the horizon.

The second of these experiments, conducted with *Shigella sonnei,* revealed that the reflective plastic pouch continued to be effective against potential diarrheal agents, even with a further decline in available sunlight as evidenced by an average external temperature of 10°C. While viable bacteria contained within the reflective pouch declined 4.5 logs in 6 hours, those in the absorptive pouch were completely stable (see Figure 9). This is a strong indication that doubling the light path by incorporating a reflective backing, as opposed to an increase in temperature through absorption, is a key factor in bacterial inactivation.

A final set of experiments was conducted to evaluate the critical features of solar disinfection using the reflective plastic pouch. Reflective plastic pouches containing 1.0 liter aliquots of water were inoculated with the target microorganism *E. coli* ATCC 25922 and placed in the following manner: 1) in a three-sided cardboard box (nonreflective); a three-sided cardboard box lined with aluminum foil (reflective); 3) unprotected on the flat surface of the roof (unprotected); or 4) shielded from the sun (control). In order to evaluate the potential effects of oxidation, a fifth sample was treated by the addition of β-carotene and placed within a reflective box.

During a 6 hour exposure, viable *E. coli* counts declined almost 7.0 logs when the reflective pouch was placed within a reflective box to shield it from the cooling effects of wind and to further intensify light exposure (see Figure 10). Comparable effects were obtained with the reflective pouch was placed on the flat surface of the roof without added protection and reflective capacity. These results simplify the design of the solar disinfection system that could ultimately be employed as no additional packaging material or concepts need be incorporated.

It is interesting to note that shielding the plastic pouch in a cardboard box actually reduced the efficiency of disinfection by almost 3.0 logs. This is most likely due to the absorption of scattered light that would otherwise have reached the pouch. Lastly, the addition of β-carotene reduced the effectiveness of the solar disinfection system by almost 6.0 logs. These results could be accounted for by the antioxidative activity (quenching effect) of β-carotene or more simply by the absorbing capacity of the material in water. Additional experiments would be required to evaluate these alternatives.

Taken together, these results show that when a clean water supply is inoculated with pathogenic strains these can be significantly reduced using a freely available resource (sunlight) and a specifically designed plastic pouch constructed of materials carefully selected to transmit and amplify solar radiation. Since the system was functional during the cool weather of New England (Connecticut) at a time of declining total sunlight (Fall), it is highly likely that the system will function at least as well, if not better, in climates of higher average available sunlight. These are the developing regions where water quality is consistently poor, and the risk to infant health greatest.

The applications for such a solar disinfection system could include treatment when other options (resources) are not readily available, for example, disaster relief where widespread destruction of water treatment facilities has taken place (such as following Hurricanes or Earthquakes), and even potential military applications (individual water treatment during deployment).

It should be understood that various changes and modifications to the presently preferred embodiments described herein will be apparent to those skilled in the art. Such changes and modifications can be made without departing from the spirit and scope of the present invention and without diminishing its attended advantages. It is therefore intended that such changes and modifications be covered by the appended claims.

## Claims

1. A container for use in reducing microbial contamination in water comprising:
a body having a first and a second side and defining an interior for receiving a fluid;
the first side being defined, at least in part, by a UV transparent layer; and
the second side being defined, at least in part, by a second layer that amplifies the effect of solar energy that passes through the first side.

2. The container of Claim I wherein the container includes a member for receiving the fluid.

3. The container of Claim 2 wherein the member is a resealable fitment.

4. The container of Claim 1 wherein the second layer is constructed from a reflective plastic.

5. The container of Claim 1 wherein the second layer is constructed from a metalized reflective plastic.

6. The container of Claim 1 wherein the second layer is constructed from a material that absorbs solar radiation.

7. The container of Claim 1 wherein the second layer has a black surface.

8. The container of Claim 1 wherein the second layer is not transparent to UV light.

9. The container of Claim 1 wherein the body is made of a flexible plastic.

10. A method for reducing the bacteria contaminants in water comprising the steps of:
providing a container including a body having a first side and a second side and defining an interior therein, the first side being defined, at least in part, by a UV transparent layer and the second layer being defined, at least in part, by a layer that amplifies the effect of UV light that passes through the first side;
placing water in the container; and
exposing the container to sunlight.

11. The method of Claim 10 wherein the second layer generates heat in response to UV light.

12. The method of Claim 10 wherein the second layer reflects UV light that passes through the first layer.

13. The method of Claim 10 wherein the container is exposed to sunlight for at least five hours.

14. A container for reducing bacterial content of water comprising:
a body defining a chamber designed to receive water;
a resealable opening for receiving water;
a first layer that is transparent to at least UV light; and
a second layer that generates heat in response to UV light.

15. The container of Claim 14 wherein the second layer is constructed from a material that absorbs solar radiation.

16. The container of Claim 14 wherein the second layer has a black surface.

17. A container for reducing bacterial content of water comprising:
a body defining a chamber designed to receive water;
a resealable opening for receiving water;
a first layer that is transparent to at least UV light; and
a second layer that reflects UV light that passes through the first layer back into the chamber.

18. The container of Claim 17 wherein the second layer is constructed from a reflective plastic.

19. The container of Claim 17 wherein the second layer is constructed from a metalized reflective plastic.

20. The container of Claim 17 wherein the member is a resealable fitment.
